# EUROPÄISCHE PATENTSCHRIFT

(11) **EP 2 566 420 B1**
(45) Veröffentlichungstag und Bekanntmachung des Hinweises auf die Patenterteilung: **01.10.2014**
(21) Anmeldenummer: 11719158.5
(22) Anmeldetag: 24.03.2011
(51) Int. Cl.: A61F 5/01, A61F 13/08, A61F 13/06

(54) **TEXTILBANDAGE**
TEXTILE BANDAGE
BANDAGE TEXTILE

(30) Priorität: 07.05.2010 DE 102010020069
(43) Veröffentlichungstag der Anmeldung: 13.03.2013
(73) Patentinhaber: Otto Bock HealthCare GmbH, 37115 Duderstadt (DE)
(72) Erfinder: VOLLBRECHT, Matthias, 37412 Herzberg (DE)
(74) Vertreter: Stornebel, Kai
(86) Internationale Anmeldenummer: PCT/DE2011/000324
(87) Internationale Veröffentlichungsnummer: WO 2011/137883

(56) Entgegenhaltungen:
- EP-A1- 1 114 630
- WO-A1-2004/058117
- DE-A1- 10 358 146
- DE-U1-202005 015 371
- FR-A1- 2 591 474

## Beschreibung

Die Erfindung betrifft eine Textilbandage mit einem Bandagenkörper, der eine einem Bandagenträger zugewandte Innenseite und eine Außenseite aufweist.

Textilbandagen sind in der Regel schlauchförmige Textilerzeugnisse, die über Gliedmaßen oder den Rumpf gezogen werden und an der dafür vorgesehenen Stelle anliegen. Häufig werden Textilbandagen im Bereich von Gelenken getragen, um diese zu unterstützen oder zu schützen, beispielsweise bei Knie- oder Ellenbogenschonern im Sport oder bei Knöchelbandagen, die schützend und unterstützend wirken sollen. Darüber hinaus sind Bandagen bekannt, die um Muskelgruppen herum angeordnet sind, um neben einer Schutzfunktion eine Wärmefunktion und eine Kompressionsfunktion auszuüben. Um das Anziehen zu erleichtern und eine Anpassung an die unterschiedlichen Abmessungen der jeweiligen Körperglieder zu erreichen, sind die Textilbandagen mit einem elastischen Bandagenkörper ausgestattet, der es erlaubt, dass nach einer Aufweitung die Bandage an dem dafür vorgesehenen Ort eng anliegt, ohne den Blutkreislauf zu stören.

Während des Tragens einer Bandage kommt es aufgrund der Bewegung der Gliedmaßen oder des Rumpfes zu einer Relativbewegung zwischen der Bandage und der Gliedmaße, so dass Bandagen häufig verrutschen. Dies wird regelmäßig dadurch ausgeglichen, dass die Bandagen von dem Bandagenträger neu positioniert werden. Ist die Bandage verrutscht, kann die vorgesehene Funktion des Schutzes oder der Unterstützung nicht vollständig ausgeführt werden.

Die FR 2,591,474 A1 betrifft eine rohrförmige, gewebte oder gestrickte, in eine oder mehrere Richtungen verformbare Umhüllung mit zwei Lagen, mit einer äußeren Lage, die stabilisierend wirkt und einer inneren Lage, die aus einem Weichmaterial besteht.

Die WO 2004/058117 A1 betrifft ein Fasergewebe mit im Wesentlichen zufällig orientierten Fasern mit einem ersten Bereich und einer Vielzahl von davon verschiedenen zweiten Bereichen, wobei zumindest ein Bereich eine lineare Ausrichtung aufweist und eine Längsachse ausbildet und der zweite Bereich sich von dem ersten Bereich weg erstreckt.

Die EP 1 114 630 A1 betrifft eine Gelenkbandage zum Überziehen aus einem elastischen Schlauch mit einer Vorderseite und einer dieser gegenüberliegenden Beugeseite, in der ein zweilagiges, feuchteleitendes Abstandsgewirk konfektioniert ist.

Die DE 103 58 146 A1 betrifft eine elastische Bandage mit einer Vorderseite und einer dieser gegenüberliegenden Beugeseite, wobei die Beugeseite zumindest bereichsweise zweilagig ausgebildet ist. Der zweilagige Bereich ist durch ein schlauchartiges Grundgestrick ausgebildet.

Die DE 20 2005 015 371 U1 betrifft eine Gelenkbandage aus einem als Flachgestrick ausgebildeten Grundgestrick, in dem in einem Gelenkbereich nahtlos ein Einsatz eingearbeitet ist. Der Einsatz ist von einem mehrlagigen Abstandsgetrick gebildet, das eine Innenlage und eine Außenlage sowie dazwischen angeordnete Abstandsfäden aufweist.

Aufgabe der vorliegenden Erfindung ist es, eine Textilbandage bereitzustellen, die bezüglich einer Verrutschsicherung verbesserte Eigenschaften aufweist.

Erfindungsgemäß wird diese Aufgabe durch eine Textilbandage mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Die erfindungsgemäße Textilbandage mit einem Bandagenkörper, der eine einem Bandagenträger zugewandte Innenseite und eine Außenseite aufweist, sieht vor, dass die Oberfläche der Innenseite Zonen mit unterschiedlichen Strichorientierungen aufweist. Oberflächen mit einem Strich haben die Eigenschaft, dass die Widerstände gegen eine Scherbelastung in Abhängigkeit von der Bewegungsrichtung unterschiedlich groß sind. Ähnlich einer Schuppenanordnung, die je nach Strömungsrichtung einen hohen oder geringen Strömungswiderstand bietet, wird bei Textilien, Fasern oder auch Fellen der Widerstand durch eine Orientierung der Fasern, Haare, Fäden oder dergleichen bewirkt. Andere Ausgestaltungen einer Strichorientierung können ebenfalls vorgesehen sein, beispielsweise ein geschichteter Aufbau von Falten, Materialüberlappungen oder dergleichen. Dies wird als Strichorientierung bezeichnet. Eine Anordnung von Zonen mit unterschiedlichen Strichorientierungen bewirkt, dass die Textilbandage während der Bewegung keine Vorzugsrichtung für ein Verrutschen aufweist, sondern dass die rutschhemmenden Eigenschaften in unterschiedlichen Richtungen auftreten. Dadurch werden Widerstände gegen das Verrutschen in vielfältigen Orientierungen bereitgestellt, so dass die Textilbandage auch bei Belastungen mit unterschiedlichen Richtungen der Relativbewegung an dem Bandagenträger in der vorgesehenen Position verbleibt.

In den jeweiligen Zonen kann die Strichorientierung einheitlich ausgerichtet sein, so dass relativ großflächige Bereiche mit der gleichen Strichorientierung ausgestattet sind. Dadurch ist es möglich, eine Textilbandage mit relativ wenig Aufwand herzustellen. Es kann jedoch auch vorgesehen sein, dass keine groß-flächigen Zonen in der Bandage vorgesehen sind, sondern dass nur einzelne kleine Bereiche mit unterschiedlichen Strichorientierungen auf der Innenseite der Bandage angeordnet sind. Unterschiedliche Strichorientierungen können somit unmittelbar aneinander angrenzen oder räumlich voneinander getrennt nebeneinander vorhanden sein.

Es ist nicht notwendig, dass die Zonen mit unterschiedlichen Strichorientierungen vollflächig ausgebildet sind. Ebenfalls können Bereiche ohne einen Strich ausgebildet sein, beispielsweise wenn zur Bereitstellung von besonderen Funktionen oder Aufnahmen für Funktionselemente Bereiche der Textilbandage eine abweichende Ausgestaltung aufweisen müssen. Bereiche ohne einen Strich können beispielsweise besondere Elastizitäten aufweisen, um die Textilbandage an den jeweiligen Einsatzzweck anzupassen.

Zumindest eine Zone der Textilbandage kann eine Strichorientierung aufweisen, die gegen eine Rotationsbewegung gerichtet ist, so dass die Ortsbeständigkeit der angelegten Textilbandage gewährleistet ist. Die Verdrehrichtung der Bandage ist dabei abhängig von dem individuellen Muskelaufbau, dem Muskeltonus und anderen anatomischen Gegebenheiten. Es kann für einen Therapieverlauf oder eine verbesserte Funktion notwendig und entscheiden sein, dass die Textilbandage sich nicht verdreht oder verrutscht. Daher kann zum Beispiel eine obere Zone der Textilbandage kann eine Strichorientierung aufweisen, die gegen eine Medialverdrehung gerichtet ist. Als obere Zone wird derjenige Bereich einer Textilbandage angesehen, der bei einer angelegten Textilbandage proximal orientiert ist. Um einer Rotationsbewegung der Textilbandage an dem Körper zu vermeiden, ist die Strichorientierung gegen eine Medialverdrehung orientiert, so dass ein erhöhter Widerstand gegen eine Verdrehung in diese Richtung vorhanden ist. Eine untere Zone, also eine distal orientierte Zone, kann eine Strichorientierung aufweisen, die gegen eine Lateralverdrehung gerichtet ist, also eine Verdrehung nach außen. Insbesondere eine Kombination einer Strichorientierung gegen eine Medialverdrehung mit einer Strichorientierung gegen eine Lateralverdrehung, die in unterschiedlichen Zonen angeordnet sind, führt zu einer gegenläufigen Widerstandsorientierung und zu einer Aufhebung der Verdrehungsneigung, so dass die Textilbandage eine geringere Verrutschneigung aufweist. Es kann auch vorgesehen sein, dass die obere Zone gegen eine Lateralverdrehung und die untere Zone gegen eine Medialverdrehung orientiert ist. Auch ist die Orientierung gegen eine Verdrehung um die Längserstreckung in einem Mittelbereich der Textilbandage möglich.

Es kann zumindest eine weitere Zone vorhanden sein, die eine Strichorientierung aufweist, die nur gegen ein Herunterrutschen gerichtet ist. Aufgrund der in der Regel konischen Ausgestaltung der Gliedmaßen, die in Richtung auf die Gelenke sich verjüngen, besteht die grundsätzliche Tendenz, dass Textilbandagen herunterrutschen oder im Bereich der Gelenke gestaucht werden. Eine Strichorientierung, die gegen ein Herunterrutschen oder Heraufrutschen gerichtet ist, wirkt dieser Tendenz entgegen.

Neben einer Orientierung, die gegen eine Verdrehung oder gegen ein Herauf- oder Herunterrutschen orientiert ist, können auch Strichorientierungen vorhanden sein, die einander überlagerten Bewegungskomponenten entgegenwirkt. So kann beispielsweise eine Strichorientierung, die hauptsächlich gegen eine Medial- oder Lateralverdrehung gerichtet ist, auch einen Anteil gegen ein Herunterrutschen aufweisen. Dies kann durch eine um einen gewissen Winkel verdrehte Anordnung der Strichorientierung relativ zu der Vorzugsrichtung geschehen. Dadurch wird ein Verrutschen in Umfangsrichtung und ein Herunterrutschen gleichermaßen verhindert, wobei die Widerstände gegen ein Verrutschen in die jeweilige Richtung in Abhängigkeit von der Winkelstellung der Strichorientierung variieren.

An dem Bandagenkörper können Funktionselemente angeordnet sein, beispielsweise zum Fixieren oder Orientieren von Führungen. Die Funktionselemente können als eine Patellaführung oder zur Stabilisierung des Bandagenkörpers insgesamt dienen. Durch die Anordnung von Funktionselementen kann die Bandage als eine Orthese ausgebildet sein, die eine stützende Funktion ausübt. Teile der Orthesenschiene oder die Orthesenschienen selbst können an dem Bandagenkörper selbst festgelegt werden, um eine Zuordnung der Funktionselemente zu den jeweiligen Gliedmaßen zu gewährleisten. Zur sicheren Fixierung können Zugelemente an dem Bandagenkörper oder Funktionselementen befestigt sein, beispielsweise Gurte oder Riemen, die über Schnallen oder Klettverschlüsse festgelegt werden.

Der Bandagenkörper ist vorzugsweise elastisch ausgebildet und kann aus einem Gewebe, Gewirke oder Gestrick ausgebildet sein.

Die Textilbandage ist bevorzugt als eine Kniegelenksbandage mit einer Ausnehmung oder Ausformung im Bereich der Patella ausgebildet, um so eine möglichst geringe Druckbelastung auf die Patella während der Bewegung auszuüben. Ebenfalls ist es möglich, dass bei einer Ausgestaltung der Textilbandage als Ellenbogenbandage eine Ausformung oder Ausnehmung im Bereich des Ellenbogens ausgebildet ist.

Zur Bereitstellung zur Strichorientierungen kann auf der Innenseite des Bandagenkörpers Strichvelour angeordnet sein, der ganzflächig oder in Teilstücken, die beabstandet zueinander ausgebildet sind, auf der Innenseite angeordnet ist.

Nachfolgend werden Ausführungsbeispiele der Erfindung anhand der beigefügten Figuren näher erläutert. Es zeigen:
- Figur 1: - eine Darstellung einer angelegten Kniebandage;
- Figur 2: - einen Zuschnitt einer Kniebandage gemäß Figur 1;
- Figur 3: - eine Variante der Figur 1; sowie
- Figur 4: - einen Zuschnitt der Bandage gemäß Figur 3.

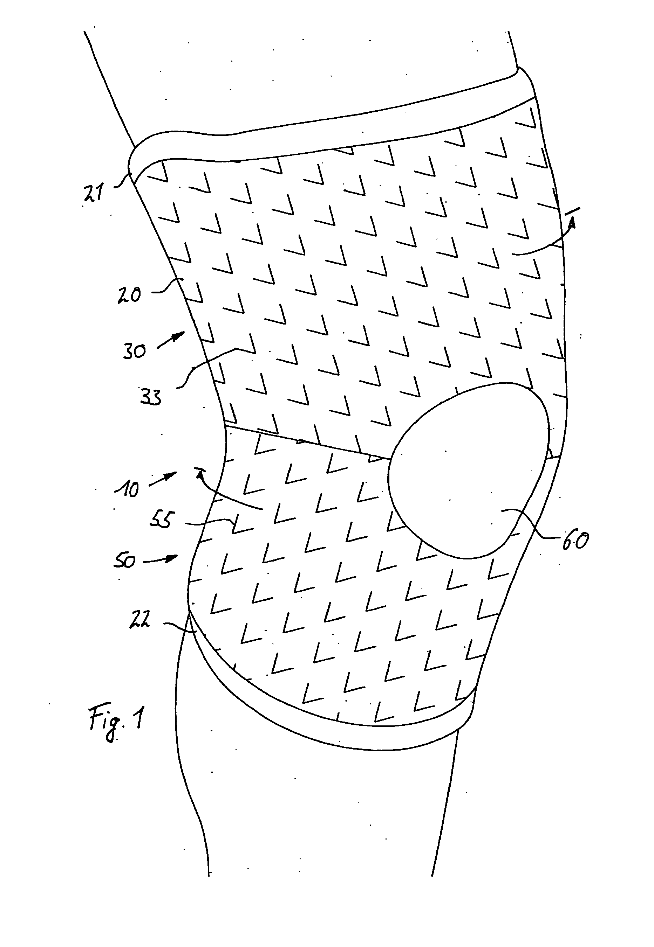

In der Figur 1 ist eine Textilbandage 10 in Gestalt einer Kniebandage dargestellt. Die Kniebandage 10 weist einen Bandagenkörper 20 auf, der schlauchförmig ausgebildet ist. An seinem proximalen Ende ist ein oberer Abschlussbund 21 angeordnet, an seinem distalen Ende ein unterer Abschlussbund 22. Der Bandagenkörper 20 ist ebenso wie die Abschlussbünde 21, 22 elastisch ausgebildet und kann als Gewebe, Gewirke oder Gestrick ausgebildet sein. Im Bereich der Patella ist eine Ausnehmung 60 ausgebildet, um die Patella während des Tragens der Textilbandage 10 nicht mit einem zu großen Druck zu beaufschlagen.

In dem dargestellten Ausführungsbeispiel ist der Bandagenkörper 20 in zwei Zonen 30, 50 aufgeteilt, eine erste Zone 30 erstreckt sich oberhalb des Kniegelenkspaltes, eine zweite Zone 50 erstreckt sich unterhalb des Kniegelenkspaltes, so dass sich eine obere oder proximale Zone 30 und eine untere oder distale Zone 50 ausbildet. Auf der Innenseite des Bandagenkörpers 20 sind Elemente mit einer Struktur aufgebracht, die unterschiedliche Strichorientierungen 33, 55 aufweisen. Die unterschiedlichen Strichorientierungen 33, 55 auf der Oberfläche der Innenseite des Bandagenkörpers 20 sind durch die jeweilig anders orientierten Muster zeichnerisch dargestellt, wobei die Strichorientierung von der offen Seite des Dreiecks zur Spitze verläuft, so dass einer Bewegung von der Spitze zur offenen Seite ein größerer Widerstand entgegensteht als umgekehrt.

In der Figur 1 ist zu erkennen, dass die Strichorientierung 33 der oberen Zone 30 der Strichorientierung 55 der unteren Zone im Wesentlichen entgegengesetzt ist. Während die Strichorientierung 33 einer Medialverdrehung entgegenwirkt, wirkt die Strichorientierung 55 der unteren Zone 50 einer Lateralverdrehung entgegen, was durch die jeweiligen Pfeile angedeutet ist.

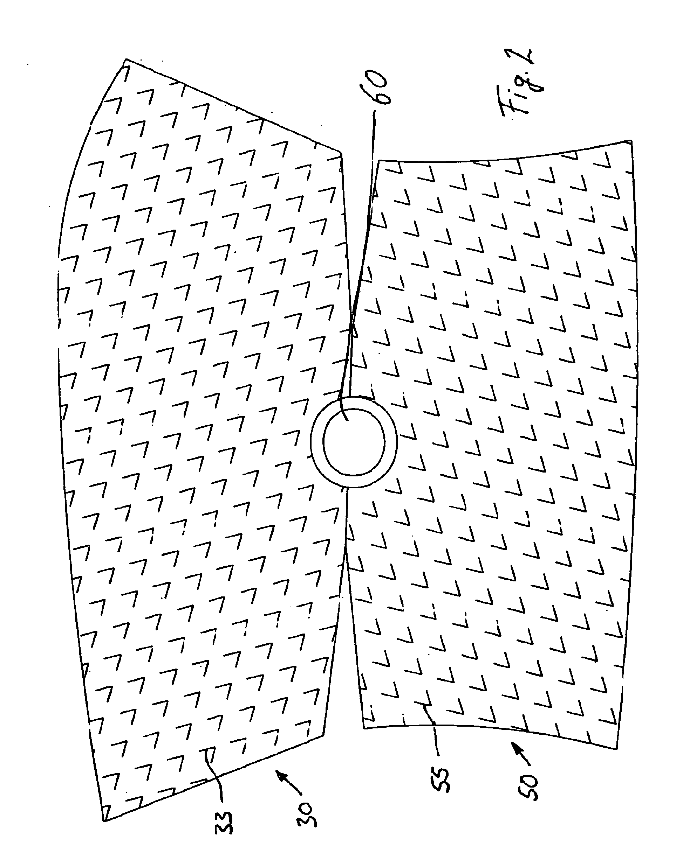

Ein Zuschnitt einer Textilbandage im nicht zusamnmengenähten Zustand ist in der Figur 2 in Draufsicht dargestellt. Zentral angeordnet ist die Ausnehmung 60 für die Patella, die Zonen 30, 50 sind vollflächig mit einem entsprechenden Material belegt, so dass einander entgegengesetzte Strichorientierungen 33, 55 vorhanden sind. Alternativ zu der dargestellten Ausführungsform mit einer vollflächigen Beschichtung oder Belegung der Oberfläche der Innenseite des Zuschnittes können auch nur einzelne Bereiche der Zonen 30, 50 mit Materialien versehen sein, die einen Strich aufweisen, so dass Bereiche ohne einen Strich sich mit Bereichen mit einem Strich abwechseln. Innerhalb der Zonen 30, 50 können auch unterschiedliche Strichorientierungen auftreten, so dass eine verbesserte Fixierung der angelegten Textilbandage erzielt wird.

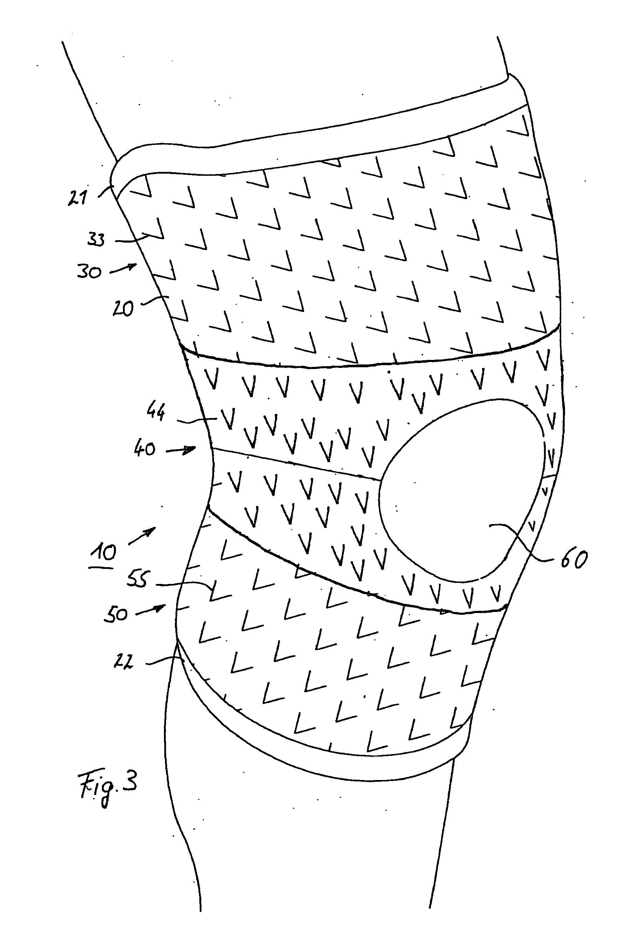

Eine Variante der Erfindung ist in der Figur 3 gezeigt, bei der die Textilbandage 10 insgesamt drei Zonen 30, 40, 50 aufweist, wobei die obere Zone 30 eine Strichorientierung 33 aufweist, die ca. 45° zur Senkrechten verdreht orientiert ist und gegen eine Verdrehung und ein Herunterrutschen orientiert ist, die mittlere Zone 40, die im Bereich der Kniekehle und der Patella ausgebildet ist, eine Strichorientierung aufweist, die nur gegen ein Herunterrutschen orientiert ist, und die untere Zone 50 eine Strichorientierung 55 aufweist, die wiederum um 45° gegenüber der Senkrechten verdreht ist und ein Herunterrutschen und eine Verdrehung verhindert oder vermindert. Die Strichorientierungen 33, 55 der oberen und unteren Zone 30, 50 sind unterschiedlich orientiert, beispielsweise einander entgegengesetzt, grundsätzlich ist es auch möglich, dass diese Orientierungen 33, 55 auch so orientiert sind, dass sie teilweise gegen ein Verrutschen, beispielsweise ein Heraufrutschen der unteren Zone oder ein Herunterrutschen der oberen Zone 30, und gegen eine Verdrehung oder nur gegen eine Verdrehung wirken.

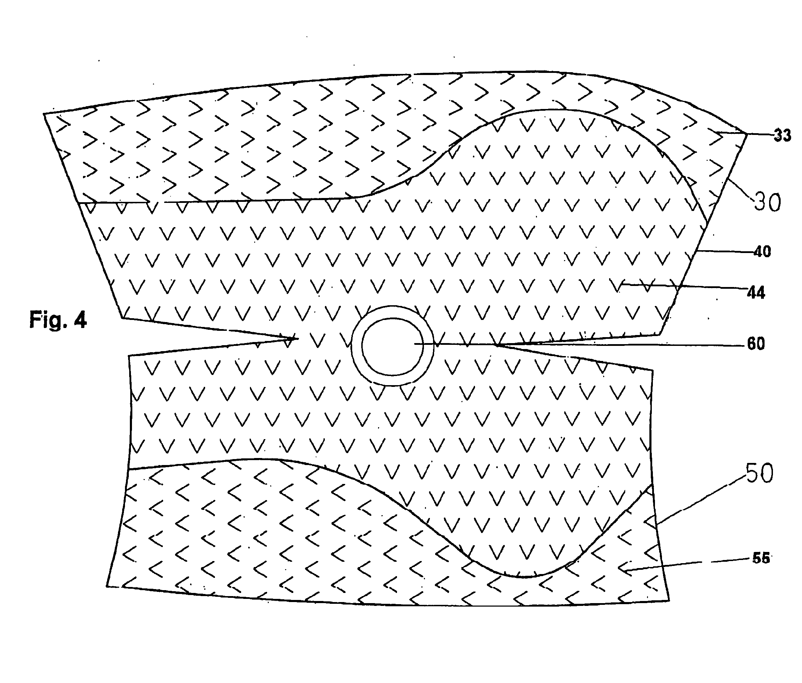

Ein möglicher Zuschnitt einer Textilbandage mit drei Zonen 30, 40, 50 ist in der Figur 4 gezeigt. Die einzelnen Zonen 30, 40, 50 sind mit geschwungenen Grenzen versehen, so dass an unterschiedlichen Stellen der Textilbandage 10 unterschiedlich große Bereiche mit entsprechenden Strichorientierungen 33, 44, 55 vorhanden sind. Dadurch kann sichergestellt werden, dass diejenigen Bereiche, die einer hohen Verdrehung oder Scherbelastung ausgesetzt sind, einen besonders hohen Verdrehungswiderstand durch eine entsprechend große Fläche entsprechend ausgebildeter Strichorientierungen aufweisen.

Die Strichorientierungen 33, 44, 55 können beispielsweise durch ein Strichvelour oder eine andere Oberflächengestaltung realisiert werden. Wesentlich dabei ist, dass unterschiedliche Scherwiderstände in Abhängigkeit von der Bewegungsrichtung zur Verfügung gestellt werden, so dass in einer Richtung ein erhöhter Widerstand und in der entgegengesetzten Richtung ein verringerter Widerstand bereitgestellt wird. Die Strichorientierungen der oberen und unteren Zone 30, 50 sind entgegengesetzt und gegen eine Verdrehung gerichtet, die mittlere Zone 40 sieht eine parallel zur Senkrechten gerichtete Orientierung vor, die gegen ein Verrutschen in Längsrichtung der Gliedmaße oder der Textilbandage wirkt.

Die Strichorientierungen 33, 55 in den oberen und unteren Zonen 30, 50 können gegenläufig ausgebildet sein, um ein Verdrehen zu vermeiden, damit Funktionselemente, die an der Textilbandage 10 angeordnet sein können, an Ort und Stelle bleiben. Der Zuschnitt gemäß Figur 4 ist für eine Kniebandage geeignet, da die obere Zone 30 breiter als die untere Zone 50 ist und somit den anatomischen Gegebenheiten des Knies mit Oben- und Unterschenkel angepasst ist.

## Patentansprüche

1. Textilbandage mit einem Bandagenkörper (20), der eine einem Bandagenträger zugewandte Innenseite und eine Außenseite aufweist, **dadurch gekennzeichnet, dass** die Oberfläche der Innenseite Zonen (30, 40, 50) mit unterschiedlichen Strichorientierungen (33, 44, 55) aufweist.

2. Textilbandage nach Anspruch 1, **dadurch gekennzeichnet, dass** in den Zonen (30, 40, 50) die Strichorientierung (33, 44, 55) einheitlich ausgerichtet ist.

3. Textilbandage nach Anspruch 1 oder 2, **dadurch gekennzeichnet, dass** in den Zonen (30, 40, 50) Bereiche ohne einen Strich ausgebildet sind.

4. Textilbandage nach einem der voranstehenden Ansprüche, **dadurch gekennzeichnet, dass** zumindest eine Zone (30, 50) eine Strichorientierung (33, 55) aufweist, die gegen eine Rotationsbewegung gerichtet ist.

5. Textilbandage nach Anspruch 4, **dadurch gekennzeichnet, dass** die Strichorientierung (33, 55) gegen eine Verdrehung einen Orientierungsanteil gegen Herunterrutschen oder Heraufrutschen aufweist.

6. Textilbandage nach einem der voranstehenden Ansprüche, **dadurch gekennzeichnet, dass** eine Zone (40) eine Strichorientierung (44) aufweist, die gegen ein Herunterrutschen oder Heraufrutschen gerichtet ist.

7. Textilbandage nach einem der voranstehenden Ansprüche, **dadurch gekennzeichnet, dass** an den Bandagenkörper (20) Funktionselemente angeordnet sind.

8. Textilbandage nach einem der voranstehenden Ansprüche, **dadurch gekennzeichnet, dass** der Bandagenkörper (20) elastisch ausgebildet ist.

9. Textilbandage nach einem der voranstehenden Ansprüche, **dadurch gekennzeichnet, dass** sie als Kniegelenksbandage mit einer Ausnehmung (60) oder Ausformung im Bereich der Patella oder als Ellenbogenbandage mit einer Ausnehmung (60) oder Ausformung im Bereich des Ellenbogens ausgebildet ist.

10. Textilbandage nach einem der voranstehenden Ansprüche, **dadurch gekennzeichnet, dass** auf der Innenseite des Bandagenkörpers (20) Strichvelours angeordnet ist.

## Claims

1. A textile bandage with a bandage body (20) which has an inner face, directed toward a bandage wearer, and an outer face, **characterized in that** the surface of the inner face has zones (30, 40, 50) with different nap orientations (33, 44, 55).

2. The textile bandage as claimed in claim 1, **characterized in that** the nap orientation (33, 44, 55) is configured uniformly in the zones (30, 40, 50).

3. The textile bandage as claimed in claim 1 or 2, **characterized in that** areas without a nap are formed in the zones (30, 40, 50).

4. The textile bandage as claimed in one of the preceding claims, **characterized in that** at least one zone (30, 50) has a nap orientation (33, 55) directed against a rotation movement.

5. The textile bandage as claimed in claim 4, **characterized in that** the nap orientation (33, 55) against rotation has an orientation component against downward shifting or upward shifting.

6. The textile bandage as claimed in one of the preceding claims, **characterized in that** a zone (40) has a nap orientation (44) directed against downward shifting or upward shifting.

7. The textile bandage as claimed in one of the preceding claims, **characterized in that** functional elements are arranged or the bandage body (20).

8. The textile bandage as claimed in one of the preceding claims, **characterized in that** the bandage body (20) is elastic.

9. The textile bandage as claimed in one of the preceding claims, **characterized in that** it is designed as a knee-joint bandage with a recess (60) or cutout in the area of the patella or as an elbow bandage with a recess (60) or cutout in the area of the elbow.

10. The textile bandage as claimed in one of the preceding claims, **characterized in that** nap velour is arranged on the inner face of the bandage body (20).

## Revendications

1. Bandage textile comprenant un corps de bandage (20) qui présente une face intérieure tournée vers un porte-bandage et une face extérieure, **caractérisé en ce que** la surface de la face intérieure comprend des zones (30, 40, 50) avec différentes orientations des raies (33, 44, 55).

2. Bandage textile selon la revendication 1, **caractérisé en ce que** l'orientation des raies (33, 44, 55) est dirigée de façon unitaire à l'intérieur des zones (30, 40, 50)

3. Bandage textile selon la revendication 1 ou 2, **caractérisé en ce que** des régions dépourvues de raies sont réalisées dans les zones (30, 40, 50).

4. Bandage textile selon l'une des revendications précédentes, **caractérisé en ce qu'**au moins une zone (30, 50) présente une orientation des raies (33, 55) qui est orientée en sens contraire un mouvement de rotation.

5. Bandage textile selon la revendication 4, **caractérisé en ce que** l'orientation des raies (33, 55), s'opposent à une torsion, présente une part d'orientation destinée à s'opposer à un glissement descendant ou montant.

6. Bandage textile selon l'une des revendications précédentes, **caractérisé en ce qu'**une zone (40) présente une orientation des raies (44) qui est dirigée pour s'opposer à un glissement descendant ou montant.

7. Bandage textile selon l'une des revendications précédentes, **caractérisé en ce que** des éléments fonctionnels sont agencés sur le corps de bandage (20).

8. Bandage textile selon l'une des revendications précédentes, **caractérisé en ce que** le corps de bandage (20) est réalisé élastique.

9. Bandage textile selon l'une des revendications précédentes, **caractérisé en ce qu'**il est réalisé à titre de bandage pour l'articulation du genou avec un évidement (60) ou un bombement dans la région de la patella, ou à titre de bandage pour le coude avec un évidement (60) ou un bombement dans la région du coude.

10. Bandage textile selon l'une des revendications précédentes, **caractérisé en ce qu'**un velours rayé est agencé sur la face intérieure du corps de bandage.
